# EUROPEAN PATENT SPECIFICATION

(11) **EP 1 080 227 B1**
(45) Date of publication and mention of the grant of the patent: **08.02.2006**
(21) Application number: 99924210.0
(22) Date of filing: 12.05.1999
(51) Int. Cl.: C12Q 1/68, G01N 33/53, G01N 33/574

(54) **Method of diagnosing, monitoring, and staging colon cancer**
Verfahren zur Diagnose, Erkennung, und Einstufung von Dickdarmkrebs
Procédé de diagnostic, de surveillance et de détermination du stade du cancer du colon

(30) Priority: 21.05.1998 US 86266 P
(43) Date of publication of application: 07.03.2001
(73) Proprietor: Diadexus, Inc., South San Francisco, CA 94080 (US)
(72) Inventor: MACINA, Roberto, A., San Jose, CA 95136 (US); YANG, Fei, San Diego, CA 92128 (US); SUN, Yongming, San Jose, CA 92128 (US)
(74) Representative: Ablett, Graham Keith
(86) International application number: PCT/US1999/010498
(87) International publication number: WO 1999/060161

(56) References cited:
- WO-A-96/39419
- WO-A-97/42506
- WO-A-98/44133
- WO-A-98/44159
- WO-A1-96/39419

## Description

### FIELD OF THE INVENTION

This invention relates, in part, to newly developed assays for detecting, diagnosing, monitoring, staging, and prognosticating cancers, particularly colon cancer.

### BACKGROUND OF THE INVENTION

Colon cancer is the second most frequently diagnosed malignancy in the United States. Cancer of the gastrointestinal tract, especially colon cancer, is a highly treatable and often a curable disease when localized to the bowel. However, currently colon cancer is the second most common cause of cancer death. Surgery is the primary treatment and results in cure in approximately 50% of patients. Recurrence following surgery is a major problem and often is the ultimate cause of death. The prognosis of colon cancer is clearly related to the degree of penetration of the tumor through the bowel wall and the presence or absence of nodal involvement. These two characteristics form the basis for all staging systems developed for this disease. Bowel obstruction and bowel perforation are indicators of poor prognosis. Elevated pretreatment serum levels of carcinoembryonic antigen (CEA) and carbohydrate antigen 19-9 (CA 19-9) also have negative prognostic significance.

Because of the frequency of the disease (approximately 160,000 new cases of colon cancer per year), the identification of high-risk groups, the demonstrated slow growth of primary lesions, the better survival of early-stage lesions, and the relative simplicity and accuracy of screening tests, screening for colon cancer should be a part of routine care for all adults starting at age 50, especially those with first-degree relatives with colorectal cancer.

Procedures used for detecting, diagnosing, monitoring, staging, and prognosticating colon cancer are of critical importance to the outcome of the patient. For example, patients diagnosed with early colon cancer generally have a much greater five-year survival rate as compared to the survival rate for patients diagnosed with distant metastasized colon cancer. Treatment decisions are usually made in reference to the older Dukes or the Modified Astler-Coller (MAC) classification schema for staging. However, new diagnostic methods which are more sensitive and specific for detecting early colon cancer are clearly needed.

Further, colon cancer patients must be closely monitored following initial therapy and during adjuvant therapy to determine response to therapy and to detect persistent or recurrent disease of metastasis. Thus, there is clearly a need for a colon cancer marker which is more sensitive and specific in detecting colon cancer recurrence.

Another important step in managing colon cancer is to determine the stage of the patient's disease. Stage determination has potential prognostic value and provides criteria for designing optimal therapy. Currently, pathological staging of colon cancer is preferable over clinical staging as pathological staging provides a more accurate prognosis. However, clinical staging would be preferred were the method of clinical staging at least as accurate as pathological staging because it does not depend on an invasive procedure to obtain tissue for pathological evaluation. Staging of colon cancer would be improved by detecting new markers in cells, tissues, or bodily fluids which could differentiate between different stages of invasion.

WO 96/39419 describes polynucleotides and polypeptides encoded thereby, which are useful as diagnostic markers for colon cancer and as agents to determine if colon cancer has metastasized.

In the present invention, methods are provided for detecting, diagnosing, monitoring, staging, and prognosticating colon cancer, via Colon Specific Genes (CSGs) with SEQ ID NO: 3 or 7. The CSGs refer, among other things, to native proteins expressed by the genes comprising the polynucleotide sequences of SEQ ID NO: 3 or 7. In the alternative, what is meant by the CSGs as used herein, means the native mRNAs encoded by the genes comprising any of the polynucleotide sequences of SEQ ID NO: 3 or 7 or levels of the genes comprising any of the polynucleotide sequences of SEQ ID NO: 3 or 7.

Other objects, features, advantages and aspects of the present invention will become apparent to those of skill in the art from the following description. It should be understood, however, that the following description and the specific examples, while indicating preferred embodiments of the invention, are given by way of illustration only.

### SUMMARY OF THE INVENTION

According to a first aspect of the present invention, there is provided a diagnostic method indicative of the presence of colon cancer in a patient, which method comprises measuring levels of CSG in a sample of cells, tissue or bodily fluid obtained from the patient and comparing the measured levels of CSG with levels of CSG in a sample of cells, tissue, or bodily fluid obtained from a control, wherein an increase in the measured CSG levels in the patient versus levels of CSG in the control is associated with colon cancer and wherein the CSG comprises SEQ ID NO:3 or 7.

According to another aspect of the present invention, there is provided a diagnostic method indicative of the presence of metastatic colon cancer in a patient, which method comprises measuring CSG levels in a sample of cells, tissue, or bodily fluid obtained from the patient and comparing the measured CSG levels with levels of CSG in a sample of cells, tissue, or bodily fluid obtained from a control, wherein an increase in measured CSG levels in the patient versus levels of CSG in the control is associated with a cancer which has metastasized and wherein the CSG comprises SEQ ID NO:3 or 7.

According to another aspect of the present invention there is provided a method of staging colon cancer in a patient identified as suffering from colon cancer which comprises measuring levels of CSG in a sample of cells, tissues, or bodily fluid obtained from the patient, and comparing the measured CSG levels with levels of CSG in a sample of cells, tissue or bodily fluid obtained from a control. An increase in measured CSG levels in the patient versus CSG levels in the control is associated with a cancer which is progressing while a decrease or equivalent level of CSG measured in the patient versus the control is associated with a cancer which is regressing or in remission and wherein the CSG comprises SEQ ID NO:3 or 7.

According to a further aspect of the present invention there is provided a method of monitoring colon cancer in a patient identified as having colon cancer that is not known to have metastasized for the onset of metastasis. The method comprises periodically measuring levels of CSG in a sample of cells, tissues, or bodily fluid obtained from the patient, and comparing the measured CSG levels with levels of CSG in a sample of cells, tissue, or bodily fluid obtained from a control, wherein an increase in measured CSG levels versus control CSG levels is associated with a cancer which has metastasized and wherein the CSG comprises SEQ ID NO:3 or 7.

According to another aspect of the present invention, there is provided a method of monitoring changes in a stage of colon cancer in a patient identified as suffering from colon cancer, the method comprising periodically measuring levels of CSG in a sample of cells, tissue, or bodily fluid obtained from the patient, and comparing the measured CSG levels with levels of CSG in a sample of cells, tissues, or bodily fluid type obtained from a control wherein an increase in any one of the measured CSG levels versus the control CSG levels is associated with a cancer which is progressing and a decrease in the measured CSG levels versus the control CSG levels is associated with a cancer which is regressing or in remission, and wherein the CSG comprises SEQ ID NO:3 or 7.

Preferably, in each of the aforementioned aspects of the present invention, the cells, tissue or bodily fluid obtained from the control are of the same type as the cells, tissue or bodily fluid obtained from the patient.

Other objects, features, advantages and aspects of the present invention will become apparent to those of skill in the art from the following description. It should be understood, however, that the following description and the specific examples, while indicating preferred embodiments of the invention, are given by way of illustration only.

### DESCRIPTION OF THE INVENTION

The present invention relates to diagnostic assays and methods, both quantitative and qualitative for detecting, diagnosing, monitoring, staging, and prognosticating cancers by comparing levels of CSG with those of CSG in a normal human control. What is meant by "levels of CSG" as used herein, means levels of the native protein expressed by the genes comprising the polynucleotide sequence of any of SEQ ID NO: 3 or 7. In the alternative, what is meant by "levels of CSG" as used herein, means levels of the native mRNA encoded by any of the genes comprising any of the polynucleotide sequences of SEQ ID NO: 3 or 7 or levels of the gene comprising any of the polynucleotide sequence of SEQ ID NO: 3 or 7. Such levels are preferably measured in at least one of, cells, tissues and/or bodily fluids, including determination of normal and abnormal levels. Thus, for instance, a diagnostic assay in accordance with the invention for diagnosing over-expression of any one of the CSG proteins compared to normal control bodily fluids, cells, or tissue samples may be used to diagnose the presence of cancers, including colon cancer. Any of the CSGs may be measured alone in the methods of the invention, or all together or any combination thereof.

By "control" it is meant a human patient without cancer and/or non cancerous samples from the patient, also referred to herein as a normal human control; in the methods for diagnosing or monitoring for metastasis, control may also include samples from a human patient that is determined by reliable methods to have colon cancer which has not metastasized.

All the methods of the present invention may optionally include measuring the levels of other cancer markers as well as CSG. Other cancer markers, in addition to CSG, useful in the present invention will depend on the cancer being tested and are known to those of skill in the art.

### Diagnostic Assays

The present invention provides methods for diagnosing the presence of colon cancer by analyzing for changes in levels of CSG in cells, tissues or bodily fluids compared with levels of CSG in cells, tissues or bodily fluids of preferably the same type from a normal human control, wherein an increase in levels of CSG in the patient versus the normal human control is associated with the presence of colon cancer. Without limiting the instant invention, typically, for a quantitative diagnostic assay a positive result indicating the patient being tested has cancer is one in which cells, tissues, or bodily fluid levels of the cancer marker, such as CSG, are at least two times higher, and most preferably are at least five times higher, than in preferably the same cells, tissues, or bodily fluid of a normal human control.

The present invention also provides a method of diagnosing metastatic colon cancer in a patient having colon cancer which has not yet metastasized for the onset of metastasis. In the method of the present invention, a human cancer patient suspected of having colon cancer which may have metastasized (but which was not previously known to have metastasized) is identified. This is accomplished by a variety of means known to those of skill in the art. For example, in the case of colon cancer, patients are typically diagnosed with colon cancer following traditional detection methods.

In the present invention, determining the presence of CSG level in cells, tissues, or bodily fluid, is particularly useful for discriminating between colon cancer which has not metastasized and colon cancer which has metastasized. Existing techniques have difficulty discriminating between colon cancer which has metastasized and colon cancer which has not metastasized and proper treatment selection is often dependent upon such knowledge.

In the present invention, the cancer marker levels measured in such cells, tissues, or bodily fluid is CSG, and are compared with levels of CSG in preferably the same cells, tissue, or bodily fluid type of a normal human control. That is, if the cancer marker being observed is just CSG in serum, this level is preferably compared with the level of CSG in serum of a normal human patient. An increase in the CSG in the patient versus the normal human control is associated with colon cancer which has metastasized.

Without limiting the instant invention, typically, for a quantitative diagnostic assay a positive result indicating the cancer in the patient being tested or monitored has metastasized is one in which cells, tissues, or bodily fluid levels of the cancer marker, such as CSG, are at least two times higher, and most preferable are at least five times higher, than in preferably the same cells, tissues, or bodily fluid of a normal patient.

Normal human control as used herein includes a human patient without cancer and/or non cancerous samples from the patient; in the methods for diagnosing or monitoring for metastasis, normal human control may also include samples from a human patient that is determined by reliable methods to have colon cancer which has not metastasized.

### Staging

The invention also provides a method of staging colon cancer in a human patient.

The method comprises identifying a human patient having such cancer; analyzing a sample of cells, tissues, or bodily fluid from such patient for CSG. Then, the method compares CSG levels in such cells, tissues, or bodily fluid with levels of CSG in preferably the same cells, tissues, or bodily fluid type of a normal human control sample, wherein an increase in CSG levels in the patient versus the normal human control is associated with a cancer which is progressing and a decrease in the levels of CSG is associated with a cancer which is regressing or in remission.

### Monitoring

Further provided is a method of monitoring colon cancer in a human having such cancer for the onset of metastasis. The method comprises identifying a human patient having such cancer that is not known to have metastasized; periodically analyzing a sample of cells, tissues, or bodily fluid from such patient for CSG; comparing the CSG levels in such cells, tissue, or bodily fluid with levels of CSG in preferably the same cells, tissues, or bodily fluid type of a normal human control sample, wherein an increase in CSG levels in the patient versus the normal human control is associated with a cancer which has metastasized.

Further provided by this inventions is a method of monitoring the change in stage of colon cancer in a human having such cancer. The method comprises identifying a human patient having such cancer; periodically analyzing a sample of cells, tissues, or bodily fluid from such patient for CSG; comparing the CSG levels in such cells, tissue, or bodily fluid with levels of CSG in preferably the same cells, tissues, or bodily fluid type of a normal human control sample, wherein an increase in CSG levels in the patient versus the normal human control is associated with a cancer which is progressing in stage and a decrease in the levels of CSG is associated with a cancer which is regressing in stage or in remission.

Monitoring such patient for onset of metastasis is periodic and preferably done on a quarterly basis. However, this may be more or less frequent depending on the cancer, the particular patient, and the stage of the cancer.

### Assay Techniques

Assay techniques that can be used to determine levels of gene expression, such as CSG of the present invention, in a sample derived from a host are well-known to those of skill in the art. Such assay methods include radioimmunoassays, reverse transcriptase PCR (RT-PCR) assays, immunohistochemistry assays, *in situ* hybridization assays, competitive-binding assays, Western Blot analyses and ELISA assays. Among these, ELISAs are frequently preferred to diagnose a gene's expressed protein in biological fluids. An ELISA assay initially comprises preparing an antibody, if not readily available from a commercial source, specific to CSG, preferably a monoclonal antibody. In addition a reporter antibody generally is prepared which binds specifically to CSG. The reporter antibody is attached to a detectable reagent such as radioactive, fluorescent or enzymatic reagent, for example horseradish peroxidase enzyme or alkaline phosphatase.

To carry out the ELISA, antibody specific to CSG is incubated on a solid support, e.g., a polystyrene dish, that binds the antibody. Any free protein binding sites on the dish are then covered by incubating with a non-specific protein such as bovine serum albumin. Next, the sample to be analyzed is incubated in the dish, during which time CSG binds to the specific antibody attached to the polystyrene dish. Unbound sample is washed out with buffer. A reporter antibody specifically directed to CSG and linked to horseradish peroxidase is placed in the dish resulting in binding of the reporter antibody to any monoclonal antibody bound to CSG. Unattached reporter antibody is then washed out. Reagents for peroxidase activity, including a colorimetric substrate are then added to the dish. Immobilized peroxidase, linked to CSG antibodies, produces a colored reaction product. The amount of color developed in a given time period is proportional to the amount of CSG protein present in the sample. Quantitative results typically are obtained by reference to a standard curve.

A competition assay may be employed wherein antibodies specific to CSG attached to a solid support and labeled CSG and a sample derived from the host are passed over the solid support and the amount of label detected attached to the solid support can be correlated to a quantity of CSG in the sample. Nucleic acid methods may be used to detect CSG mRNA as a marker for colon cancer. Polymerase chain reaction (PCR) and other nucleic acid methods, such as ligase chain reaction (LCR) and nucleic acid sequence based amplification (NASABA), can be used to detect malignant cells for diagnosis and monitoring of various malignancies. For example, reverse-transcriptase PCR (RT-PCR) is a powerful technique which can be used to detect the presence of a specific mRNA population in a complex mixture of thousands of other mRNA species. In RT-PCR, an mRNA species is first reverse transcribed to complementary DNA (cDNA) with use of the enzyme reverse transcriptase; the cDNA is then amplified as in a standard PCR reaction. RT-PCR can thus reveal by amplification the presence of a single species of mRNA. Accordingly, if the mRNA is highly specific for the cell that produces it, RT-PCR can be used to identify the presence of a specific type of cell.

Hybridization to clones or oligonucleotides arrayed on a solid support (i.e., gridding) can be used to both detect the expression of and quantitate the level of expression of that gene. In this approach, a cDNA encoding the CSG gene is fixed to a substrate. The substrate may be of any suitable type including but not limited to glass, nitrocellulose, nylon or plastic. At least a portion of the DNA encoding the CSG gene is attached to the substrate and then incubated with the analyte, which may be RNA or a complementary DNA (cDNA) copy of the RNA, isolated from the tissue of interest.
Hybridization between the substrate bound DNA and the analyte can be detected and quantitated by several means including but not limited to radioactive labeling or fluorescence labeling of the analyte or a secondary molecule designed to detect the hybrid. Quantitation of the level of gene expression can be done by comparison of the intensity of the signal from the analyte compared with that determined from known standards. The standards can be obtained by *in vitro* transcription of the target gene, quantitating the yield, and then using that material to generate a standard curve.
The above tests can be carried out on samples derived from a variety of patients' cells, bodily fluids and/or tissue extracts (homogenates or solubilized tissue) such as from tissue biopsy and autopsy material. Bodily fluids useful in the present invention include blood, urine, saliva, or any other bodily secretion or derivative thereof. Blood can include whole blood, plasma, serum, or any derivative of blood.

### EXAMPLES

The present invention is further described by the following examples. These examples are provided solely to illustrate the invention by reference to specific embodiments. These exemplifications, while illustrating certain specific aspects of the invention, do not portray the limitations or circumscribe the scope of the disclosed invention.

### Example 1: CSGs

Searches were carried out and CSGs identified using the following Search Tools as part of the LIFESEQ® database available from Incyte Pharmaceuticals, Palo Alto, CA:
1. Library Comparison (compares one library to one other library) allows the identification of clones expressed in tumor and absent or expressed at a lower level in normal tissue.
2. Subsetting is similar to library comparison but allows the identification of clones expressed in a pool of libraries and absent or expressed at a lower level in a second pool of libraries.
3. Transcript Imaging lists all of the clones in a single library or a pool of libraries based on abundance. Individual clones can then be examined using Electronic Northerns to determine the tissue sources of their component ESTs.
4. Protein Function: Incyte has identified subsets of ESTs with a potential protein function based on homologies to known proteins. Some examples in this database include Transcription Factors and Proteases. We identified some leads by searching in this database for clones whose component ESTs showed disease specificity.

Electronic subtractions, transcript imaging and protein function searches were used to identify clones, whose component ESTs were exclusively or more frequently found in libraries from specific tumors. Individual candidate clones were examined in detail by checking where each EST originated.

**Table 1: CSGs**

| **SEQ ID NO:** | **Clone ID #** | **Gene ID #** | |
|---|---|---|---|
| 1 | 238330 | 242807 | Transcript Imaging |
| 2 | 1285234 | 239588 | Subsetting |
| 3 | 1341701 | 29634 | Transcript Imaging |
| 4 | 816257 | 233421 | Subsetting |
| 5 | 775133 | 245080 | Subsetting |
| 6 | 1335450 | 245811 | Subsetting |
| 7 | 2348122 | 233711 | Transcript Imaging |
| 8 | 3228674 | 230273 | Subsetting |
| 9 | 1632174 | 229022 | Transcript Imaging |

The following example was carried out using standard techniques, which are well known and routine to those of skill in the art, except where otherwise described in detail. Routine molecular biology techniques of the following example can be carried out as described in standard laboratory manuals, such as Sambrook et al., MOLECULAR CLONING: A LABORATORY MANUAL, 2nd Ed.; Cold Spring Harbor Laboratory Press, Cold Spring Harbor, N.Y. (1989).

### Example 2: Relative Quantitation of CSG Gene Expression

Real-Time quantitative PCR with fluorescent Taqman probes is a quantitation detection system utilizing the 5'-3' nuclease activity of Taq DNA polymerase. The method uses an internal fluorescent oligonucleotide probe (Taqman) labeled with a 5' reporter dye and a downstream, 3' quencher dye. During PCR, the 5'-3' nuclease activity of Taq DNA polymerase releases the reporter, whose fluorescence can then be detected by the laser detector of the Model 7700 Sequence Detection System (PE Applied Biosystems, Foster City, CA, USA).

Amplification of an endogenous control is used to standardize the amount of sample RNA added to the reaction and normalize for Reverse Transcriptase (RT) efficiency. Either cyclophilin, glyceraldehyde-3-phosphate dehydrogenase (GAPDH) or 18S ribosomal RNA (rRNA) is used as this endogenous control. To calculate relative quantitation between all the samples studied, the target RNA levels for one sample are used as the basis for comparative results (calibrator). Quantitation relative to the "calibrator" can be obtained using the standard curve method or the comparative method (User Bulletin #2: ABI PRISM 7700 Sequence Detection System).

To evaluate the tissue distribution, and the level of CSGs in normal and tumor tissue, total RNA was extracted from normal tissues, tumor tissues, and from tumors and the corresponding matched normal tissues. Subsequently, first strand cDNA was prepared with reverse transcriptase and the polymerase chain reaction was done using primers and Taqman probe specific to the CSG. The results were analyzed using the ABI PRISM 7700 Sequence Detector. The absolute numbers are relative levels of expression of the CSG compared to the calibrator.

### Comparative Examples

Similar mRNA expression analysis for genes coding for the diagnostic markers PSA (Prostate Specific Antigen) and PLA2 (Phospholipase A2) was performed for comparison. PSA is currently the only cancer screening marker available in clinical laboratories. When the panel of normal pooled tissues was analyzed, PSA was expressed at very high levels in prostate, with a very low expression in breast and testis. After analysis of more than 55 matching samples from 14 different tissues, the data corroborated the tissue specificity seen with normal tissue samples. PSA expression was compared in cancer and normal adjacent tissue for 12 matching samples of prostate tissue. The relative levels of PSA were higher in 10 cancer samples (83%). Clinical data recently obtained support the utilization of PLA2 as a staging marker for late stages of prostate cancer. mRNA expression data described herein showed overexpression of the mRNA in 8 out of the 12 prostate matching samples analyzed (66%). PLA2 had high levels of mRNA expression in small intestine, prostate, liver, and pancreas.

### Measurement of SEQ ID NO:3; Clone ID 1341701; Gene ID 29634 (Cln106)

Absolute numbers are depicted in Table 2 as relative levels of expression of Cln106 (SEQ ID NO:3) in 12 normal different tissues. All the values are compared to normal testis (calibrator). These RNA samples are commercially available pools, originated by pooling samples of a particular tissue from different individuals.

**Table 2: Relative levels of Cln106 Expression in Pooled Samples**

| **Tissue** | **NORMAL** |
|---|---|
| Colon-Ascending | 110 |
| Endometrium | 0 |
| Kidney | 0 |
| Liver | 0 |
| Ovary | 0 |
| Pancreas | 0 |
| Prostate | 16 |
| Small Intestine | 0 |
| Spleen | 0 |
| Stomach | 0 |
| Testis | 1 |
| Uterus | 0 |

The relative levels of expression in Table 2 show for the CSG Cln106 (SEQ ID NO:3), mRNA expression is more than 6 fold higher in the pool of normal ascending colon (110) compared with prostate (16). Testis, the calibrator, with a relative expression level of 1, is the only other tissue expressing the mRNA for Cln106 (SEQ ID NO:3). These results demonstrate that mRNA expression of this CSG is highly specific for colon.

The absolute numbers in Table 2 were obtained analyzing pools of samples of a particular tissue from different individuals. They can not be compared to the absolute numbers originated from RNA obtained from tissue samples of a single individual in Table 3.

The absolute numbers in Table 3 are relative levels of expression of Cln106 (SEQ ID NO:3) in 57 pairs of matching samples. All the values are compared to normal testis (calibrator). A matching pair is formed by mRNA from the cancer sample for a particular tissue and mRNA from the normal adjacent sample for that same tissue from the same individual.

**Table 3: Relative levels of Cln106 Expression in Individual Samples**

| **Sample ID** | **Tissue** | **Cancer** | **Matching Normal Adjacent** |
|---|---|---|---|
| Sto AC93 | Stomach 1 | 4 | 96 |
| Sto AC99 | Stomach 2 | 0.4 | 0.5 |
| Sml 21XA | Small Intestine 1 | 0 | 0 |
| Sml H89 | Small Intestine 2 | 0.93 | 1.28 |
| Cln B56 | Colon-Cecum(A)1 | 317 | 101 |
| Cln AS45 | Colon-Ascending(A)2 | 316.3 | 146.5 |
| Cln CM67 | Colon-Cecum(B)3 | 481.0 | 217.5 |
| Cln AS67 | Colon-Ascending(B)4 | 858.1 | 220.6 |
| Cln AS43 | Colon-Ascending(C)5 | 1370 | 98 |
| Cln AS46 | Colon-Ascending(C)6 | 3051 | 375 |
| Cln AS98 | Colon-Ascending(C)7 | 26 | 42 |
| Cln AS89 | Colon-Ascending(D)8 | 524.6 | 11.0 |
| Cln TX01 | Colon-Transverse(B)9 | 2886.3 | 1992.0 |
| Cln TX89 | Colon-Transverse(B)10 | 146.0 | 35.9 |
| Cln TX67 | Colon-Transverse(C)11 | 2.9 | 421.7 |
| Cln MT38 | Colon-Splenic Flexture(M)12 | 1681 | 187 |
| Cln SG89 | Colon-Sigmoid(B)13 | 1063.8 | 31.1 |
| Cln SG67 | Colon-Sigmoid(C)14 | 8.5 | 9.4 |
| Cln SG33 | Colon-Sigmoid(C)15 | 264 | 549 |
| Cln SG45 | Colon-Sigmoid(D)16 | 580.0 | 114.6 |
| Cln B34 | Colon-Rectosigmoid(A)17 | 97 | 244 |
| Cln CXGA | Colon-Rectum(A)18 | 45.1 | 273.4 |
| Cln RC67 | Colon-Rectum(B)19 | 2.7 | 20.0 |
| Cln C9XR | Colon-Rectosigmoid(C)20 | 609 | 460 |
| Cln RS45 | Colon-Rectosigmoid(C)21 | 472.8 | 144.0 |
| Cln RC01 | Colon-Rectum(C)22 | 568 | 129 |
| Cln RC89 | Colon-Rectum(D)23 | 4.6 | 322.91 |
| Bld 46XK | Bladder 1 | 0.2 | 0 |
| Bld 66X | Bladder 2 | 1 | 1 |
| Bld 32XK | Bladder 3 | 0.0 | 0.0 |
| Kid 126XD | Kidney 1 | 0 | 0 |
| Kid 12XD | Kidney 2 | 0 | 0 |
| Kid 5XD | Kidney 3 | 0.0 | 1.0 |
| Kid 6XD | Kidney 4 | 0.0 | 0.0 |
| Kid 106XD | Kidney 5 | 0.4 | 0.0 |
| Liv 42X | Liver 1 | 0.0 | 0.0 |
| Liv 15XA | Liver 2 | 0.0 | 0.0 |
| Liv 94XA | Liver 3 | 0.0 | 0.0 |
| Lng AC69 | Lung 1 | 2 | 0 |
| Lng BR94 | Lung 2 | 0 | 0 |
| Lng 47XQ | Lung 3 | 0 | 0 |
| Mam 59X | Mammary Gland 1 | 0 | 0 |
| Mam B011X | Mammary Gland 2 | 0 | 0 |
| Mam A06X | Mammary Gland 3 | 0 | 0 |
| Ovr 103X | Ovary 1 | 0.04 | 2.08 |
| Ovr 130X | Ovary 2 | 0.1 | 2.76 |
| Pan 71XL | Pancreas 1 | 4.08 | 0.1 |
| Pan 82XP | Pancreas 2 | 0 | 0 |
| Pro 12B | Prostate 1 | 0.3 | 0 |
| Pro 23B | Prostate 2 | 3 | 4 |
| Pro 13XB | Prostate 3 | 2 | 7 |
| Pro 34B | Prostate 4 | 0.54 | 4.01 |
| Pro 20XB | Prostate 5 | 4.8 | 4.3 |
| Pro 65XB | Prostate 6 | 0.7 | 1.3 |
| Tst 39X | Testis 1 | 2.78 | 0 |
| End 8XA | Endometrium 1 | 0 | 0.2 |
| Utr 85XU | Uterus 1 | 1.26 | 0 |

| | | | |
|---|---|---|---|
| 0= Negative | | | |

When matching samples were analyzed, the higher levels of expression were in the colon, showing a high degree of tissue specificity for this tissue. These results confirm the tissue specificity results obtained with the panel of normal pooled samples (Table 2). Furthermore, the level of mRNA expression in cancer samples and the isogenic normal adjacent tissue from the same individual were compared. This comparison provides an indication of specificity for the cancer stage (e.g. higher levels of mRNA expression in the cancer sample compared to the normal adjacent). Table 3 shows overexpression of Cln106 (SEQ ID NO:3) in 15 colon cancer tissues compared with their respective normal adjacent (colon samples #1, 2, 3, 4, 5, 6, 8, 9, 10, 12, 13, 16, 20, 21, and 22). There is overexpression in the cancer tissue for 65% of the colon matching samples tested (total of 23 colon matching samples). The matching sample Pan 71XL is a secondary cancer in pancreas, the primary cancer in that individual was a duodenal cancer.

Altogether, the high level of tissue specificity, plus the mRNA overexpression in 65% of the colon matching samples tested are demonstrative of CSG Cln106 (SEQ ID NO:3) being a diagnostic marker for colon cancer.

### Measurement of SEQ ID NO:4; Clone ID 816257; Gene ID 406452 (Cln107)

Absolute numbers as depicted in Table 4 are relative levels of expression of CSG Cln107 (SEQ ID NO:4) in 12 normal different tissues. All the values are compared to normal small intestine (calibrator). These RNA samples are commercially available pools, originated by pooling samples of a particular tissue from different individuals.

**Table 4: Relative levels of Cln107 Expression in Pooled Samples**

| **Tissue** | **NORMAL** |
|---|---|
| Colon-Ascending | 3.2 |
| Endometrium | 0 |
| Kidney | 0.2 |
| Liver | 0 |
| Ovary | 0 |
| Pancreas | 0 |
| Prostate | 0.1 |
| Small Intestine | 1 |
| Spleen | 0 |
| Stomach | 0.3 |
| Testis | 0 |
| Uterus | 0 |

The relative levels of expression in Table 4 show that mRNA expression of the CSG Cln107 (SEQ ID NO:4) is more than 10 fold higher in the pool of normal ascending colon (3.2), five fold higher in small intestine (1), and 1.5 fold higher in stomach (0.3), compared with the next higher expressor (0.2 for kidney). Seven of the pooled tissues samples analyzed were negative and prostate showed a relative expression of 0.1 for Cln107 (SEQ ID NO:4). These results demonstrate that Cln107 mRNA expression is highly specific for colon, small intestine, and in a lower degree for stomach.

The absolute numbers in Table 4 were obtained analyzing pools of samples of a particular tissue from different individuals. They can not be compared to the absolute numbers originated from RNA obtained from tissue samples of a single individual in Table 5.

The absolute numbers in Table 5 are relative levels of expression of Cln107 (SEQ ID NO:4) in 57 pairs of matching samples. All the values are compared to normal small intestine (calibrator). A matching pair is formed by mRNA from the cancer sample for a particular tissue and mRNA from the normal adjacent sample for that same tissue from the same individual.

**Table 5: Relative levels of Cln107 Expression in Individual Samples**

| **Sample ID** | **Tissue** | **Cancer** | **Matching Normal Adjacent** |
|---|---|---|---|
| Sto AC93 | Stomach 1 | 8.9 | 13.4 |
| Sto AC99 | Stomach 2 | 6.0 | 0.9 |
| Sml 21XA | Small Intestine 1 | 1.07 | 1.42 |
| Sml H89 | Small Intestine 2 | 0.97 | 4.13 |
| Cln B56 | Colon-Cecum(A)1 | 2 | 16 |
| Cln AS45 | Colon-Ascending(A)2 | 0.7 | 2.1 |
| Cln CM67 | Colon-Cecum(B)3 | 1.6 | 2.1 |
| Cln AS67 | Colon-Ascending(B)4 | 1.2 | 6.2 |
| Cln AS43 | Colon-Ascending(C)5 | 13.5 | 0.5 |
| Cln AS46 | Colon-Ascending(C)6 | 9.7 | 23.6 |
| Cln AS98 | Colon-Ascending(C)7 | 28.1 | 1.4 |
| Cln AS89 | Colon-Ascending(D)8 | 0.9 | 3.1 |
| Cln TX01 | Colon-Transverse(B)9 | 3.0 | 10.6 |
| Cln TX89 | Colon-Transverse(B)10 | 4.5 | 0.6 |
| Cln TX67 | Colon-Transverse(C)11 | 3.6 | 3.4 |
| Cln MT38 | Colon-Splenic Flexture(M)12 | 4.0 | 2.6 |
| Cln SG89 | Colon-Sigmoid(B)13 | 4.7 | 0.9 |
| Cln SG67 | Colon-Sigmoid(C)14 | 1.0 | 1.3 |
| Cln SG33 | Colon-Sigmoid(C)15 | 14.2 | 7.6 |
| Cln SG45 | Colon-Sigmoid(D)16 | 4.8 | 6.0 |
| Cln B34 | Colon-Rectosigmoid(A)17 | 3 | 2 |
| Cln CXGA | Colon-Rectum(A)18 | 4.4 | 1.9 |
| Cln RC67 | Colon-Rectum(B)19 | 0.1 | 0.4 |
| Cln C9XR | Colon-Rectosigmoid(C)20 | 5 | 3 |
| Cln RS45 | Colon-Rectosigmoid(C)21 | 11.4 | 4.6 |
| Cln RC01 | Colon-Rectum(C)22 | 1.8 | 2.3 |
| Cln RC89 | Colon-Rectum(D)23 | 0.1 | 5.35 |
| Bld 46XK | Bladder 1 | 0.2 | 0 |
| Bld 66X | Bladder 2 | 1 | 1 |
| Bld 32XK | Bladder 3 | 0.1 | 0.1 |
| Kid 126XD | Kidney 1 | 0 | 0.02 |
| Kid 12XD | Kidney 2 | 0.1 | 0.2 |
| Kid 5XD | Kidney 3 | 0.3 | 0.0 |
| Kid 6XD | Kidney 4 | 0.1 | 0.1 |
| Kid 106XD | Kidney 5 | 0.0 | 0.1 |
| Liv 42X | Liver 1 | 7.9 | 0.002 |
| Liv 15XA | Liver 2 | 0.0 | 0.0 |
| Liv 94XA | Liver 3 | 0.0 | 0.0 |
| Lng AC69 | Lung 1 | 1.6 | 0.2 |
| Lng BR94 | Lung 2 | 0.4 | 0 |
| Lng 47XQ | Lung 3 | 0.78 | 0.2 |
| Mam 59X | Mammary Gland 1 | 0.05 | 0.3 |
| Mam B011X | Mammary Gland 2 | 0.01 | 0.004 |
| Mam A06X | Mammary Gland 3 | 0.22 | 0 |
| Ovr 103X | Ovary 1 | 0.01 | 0.01 |
| Ovr 130X | Ovary 2 | 0.09 | 0.1 |
| Pan 71XL | Pancreas 1 | 2.51 | 2.81 |
| Pan 82XP | Pancreas 2 | 0 | 0.62 |
| Pro 12B | Prostate 1 | 0.3 | 0.1 |
| Pro 23B | Prostate 2 | 0.3 | 0.2 |
| Pro 13XB | Prostate 3 | 0 | 0 |
| Pro 34B | Prostate 4 | 0.04 | 0.22 |
| Pro 20XB | Prostate 5 | 0.4 | 0.1 |
| Pro 65XB | Prostate 6 | 0.0 | 0.1 |
| Tst 39X | Testis 1 | 0.02 | 0.01 |
| End 8XA | Endometrium 1 | 0.01 | 0.5 |
| Utr 85XU | Uterus 1 | 0.03 | 0 |

| | | | |
|---|---|---|---|
| 0= Negative | | | |

When matching samples were analyzed, the higher levels of expression were in colon, stomach, and small intestine, showing a high degree of tissue specificity for colon tissues. These results confirm the tissue specificity results obtained with normal pooled samples (Table 4). Furthermore, the level of mRNA expression in cancer samples and the isogenic normal adjacent tissue from the same individual were compared. This comparison provides an indication of specificity for the cancer stage (e.g. higher levels of mRNA expression in the cancer sample compared to the normal adjacent). Table 5 shows overexpression of Cln107 (SEQ ID NO:4) in 11 colon cancer tissues compared with their respective normal adjacent (colon samples #5, 7, 10, 11, 12, 13, 15, 17, 18, 20, and 21). There is overexpression in the cancer tissue for 48% of the colon matching samples tested (total of 23 colon matching samples). The matching sample Pan 71XL is a secondary cancer in pancreas, the primary cancer in that individual was a duodenal cancer.

Altogether, the high level of tissue specificity, plus the mRNA overexpression in almost half of the colon, stomach, and small intestine matching samples tested are demonstrative of CSG Cln107 (SEQ ID NO:4) being a diagnostic marker for colon cancer.

### Measurement of SEQ ID NO:5; Clone ID 775133; Gene ID 24508 (Cln108)

The absolute numbers shown in Table 6 are relative levels of expression of CSG Cln108 (SEQ ID NO:5) in 12 normal different tissues. All the values are compared to normal small intestine (calibrator). These RNA samples are commercially available pools, originated by pooling samples of a particular tissue from different individuals.

**Table 6: Relative levels of Cln108 Expression in Pooled Samples**

| **Tissue** | **NORMAL** |
|---|---|
| Colon-Ascending | 2846.5 |
| Endometrium | 1 |
| Kidney | 5.5 |
| Liver | 18.7 |
| Ovary | 3.4 |
| Pancreas | 198.1 |
| Prostate | 1024 |
| Small Intestine | 810.8 |
| Spleen | 32.2 |
| Stomach | 9981.2 |
| Testis | 0 |
| Uterus | 294.1 |

The relative levels of expression in Table 6 show that mRNA expression of CSG Cln108 (SEQ ID NO:5) is more than 10 fold higher in the pool of normal ascending colon (2846.5) and almost ten fold higher in stomach (9981.2), compared to the expression level in any other tissue analyzed. These results demonstrate that mRNA expression of this CSG is also highly specific for colon and stomach.

The absolute numbers in Table 6 were obtained analyzing pools of samples of a particular tissue from different individuals. They can not be compared to the absolute numbers originated from RNA obtained from tissue samples of a single individual in Table 7.

The absolute numbers depicted in Table 7 are relative levels of expression of Cln108 (SEQ ID NO:5) in 57 pairs of matching samples. All the values are compared to normal small intestine (calibrator). A matching pair is formed by mRNA from the cancer sample for a particular tissue and mRNA from the normal adjacent sample for that same tissue from the same individual.

**Table 7: Relative levels of Cln108 Expression in Individual Samples**

| **Sample ID** | **Tissue** | **Cancer** | **Matching Normal Adjacent** |
|---|---|---|---|
| Sto AC93 | Stomach 1 | 28696 | 34842 |
| Sto AC99 | Stomach 2 | 21523 | 30862 |
| Sml 21XA | Small Intestine 1 | 2944 | 964.4 |
| Sml H89 | Small Intestine 2 | 244.5 | 3513.2 |
| Cln B56 | Colon-Cecum(A)1 | 27242 | 24637 |
| Cln AS45 | Colon-Ascending(A)2 | 5827.0 | 8771.0 |
| Cln CM67 | Colon-Cecum(B)3 | 4251.0 | 4684.0 |
| Cln AS67 | Colon-Ascending(B)4 | 564.0 | 1949.0 |
| Cln AS43 | Colon-Ascending(C)5 | 50310 | 10949 |
| Cln AS46 | Colon-Ascending(C)6 | 246044 | 120073 |
| Cln AS98 | Colon-Ascending(C)7 | 40442 | 17482 |
| Cln AS89 | Colon-Ascending(D)8 | 5730.0 | 1581.0 |
| Cln TX01 | Colon-Transverse(B)9 | 22281.0 | 114784.0 |
| Cln TX89 | Colon-Transverse(B)10 | 11026.0 | 1639.0 |
| Cln TX67 | Colon-Transverse(C)11 | 17004.0 | 11654.0 |
| Cln MT38 | Colon-Splenic Flexture(M)12 | 77589 | 31620 |
| Cln SG89 | Colon-Sigmoid(B)13 | 140339.0 | 49617.0 |
| Cln SG67 | Colon-Sigmoid(C)14 | 4951.0 | 7905.0 |
| Cln SG33 | Colon-Sigmoid(C)15 | 60875 | 120490 |
| Cln SG45 | Colon-Sigmoid(D)16 | 30437.0 | 47267.0 |
| Cln B34 | Colon-Rectosigmoid(A)17 | 5848 | 5861 |
| Cln CXGA | Colon-Rectum(A)18 | 13877.0 | 9787.0 |
| Cln RC67 | Colon-Rectum(B)19 | 1703.0 | 26589.0 |
| Cln C9XR | Colon-Rectosigmoid(C)20 | 2458 | 19071 |
| Cln RS45 | Colon-Rectosigmoid(C)21 | 95523 | 61939 |
| Cln RC01 | Colon-Rectum(C)22 | 98891.0 | 80047.0 |
| Cln RC89 | Colon-Rectum(D)23 | 17.0 | 1775 |
| Bld 46XK | Bladder 1 | 0 | 8 |
| Bld 66X | Bladder 2 | 397 | 44 |
| Bld 32XK | Bladder 3 | 0.0 | 16.0 |
| Kid 126XD | Kidney 1 | 32 | 22 |
| Kid 12XD | Kidney 2 | 6 | 0 |
| Kid 106XD | Kidney 3 | 4.0 | 33.0 |
| Liv 42X | Liver 1 | 4783 | 0 |
| Liv 15XA | Liver 2 | 4.0 | 10.0 |
| Liv 94XA | Liver 3 | 159.0 | 21.0 |
| Lng AC69 | Lung 1 | 222 | 295 |
| Lng BR94 | Lung 2 | 112 | 0 |
| Lng 47XQ | Lung 3 | 30 | 69 |
| Lng AC66 | Lung 4 | 29 | 137 |
| Mam 59X | Mammary Gland 1 | 56 | 0 |
| Mam B011X | Mammary Gland 2 | 54 | 31 |
| Mam A06X | Mammary Gland 3 | 12 | 0 |
| Ovr 103X | Ovary 1 | 37 | 0 |
| Pan 71XL | Pancreas 1 | 13203 | 4163 |
| Pan 82XP | Pancreas 2 | 39.1 | 0 |
| Pro 12B | Prostate 1 | 386 | 88 |
| Pro 23B | Prostate 2 | 250 | 23 |
| Pro 13XB | Prostate 3 | 92 | 731 |
| Pro 34B | Prostate 4 | 33.3 | 265.7 |
| Pro 20XB | Prostate 5 | 454.6 | 1908.9 |
| Pro 65XB | Prostate 6 | 733.5 | 922.0 |
| End 8XA | Endometrium 1 | 5 | 92 |
| Utr 85XU | Uterus 1 | 98.9 | 21.8 |
| Utr 23XU | Uterus 2 | 35.3 | 0 |
| Utr 135XO | Uterus 3 | 39.2 | 43.8 |
| Utr 141XO | Uterus 4 | 212.1 | 55.9 |

| | | | |
|---|---|---|---|
| 0= Negative | | | |

When matching samples were analyzed, the higher levels of expression were in colon and stomach, showing a high degree of tissue specificity for these two tissues. These results confirm the tissue specificity results obtained with normal pooled samples (Table 6). Furthermore, the level of mRNA expression in cancer samples and the isogenic normal adjacent tissue from the same individual were compared. This comparison provides an indication of specificity for the cancer stage (e.g. higher levels of mRNA expression in the cancer sample compared to the normal adjacent). Table 7 shows overexpression of CSG Cln108 (SEQ ID NO:5) in 13 colon cancer tissues compared with their respective normal adjacent (colon samples #1, 5, 6, 7, 8, 9, 10, 11, 12, 13, 18, 21, and 22). There is overexpression in the cancer tissue for 56% of the colon matching samples tested (total of 23 colon matching samples). The matching sample Pan 71XL is a secondary cancer in pancreas, the primary cancer in that individual was a duodenal cancer.

Altogether, the high level of tissue specificity, plus the mRNA overexpression in more than half of the colon, stomach, and small intestine matching samples tested are demonstrative of this CSG, Cln108 (SEQ ID NO:5), also being a diagnostic marker for colon cancer.

### Measurement of SEQ ID NO:7; Clone ID 2348122; Gene ID 23371 (Cln109)

The absolute numbers depicted in Table 8 are relative levels of expression of CSG Cln109 (SEQ ID NO:7) in 12 normal different tissues. All the values are compared to normal ovary (calibrator). These RNA samples are commercially available pools, originated by pooling samples of a particular tissue from different individuals.

**Table 8: Relative levels of Cln109 Expression in Pooled Samples**

| **Tissue** | **NORMAL** |
|---|---|
| Colon-Ascending | 28.8 |
| Endometrium | 0.45 |
| Kidney | 0.41 |
| Liver | 0.72 |
| Ovary | 0.07 |
| Pancreas | 82.8 |
| Prostate | 124.3 |
| Small Intestine | 626.4 |
| Spleen | 1.2 |
| Stomach | 12.05 |
| Testis | 1.51 |
| Uterus | 52.99 |

The relative levels of expression in Table 8 show that mRNA expression of CSG Cln109 (SEQ ID NO:7), is more than 5 fold higher in the pool of normal small intestine (626.4) compared to the expression level in any other tissue analyzed. These results demonstrate that Cln109 (SEQ ID NO:7) mRNA expression is highly specific for small intestine

The absolute numbers in Table 8 were obtained analyzing pools of samples of a particular tissue from different individuals. They can not be compared to the absolute numbers originated from RNA obtained from tissue samples of a single individual in Table 9.

The absolute numbers depicted in Table 9 are relative levels of expression of Cln109 (SEQ ID NO:7) in 53 pairs of matching samples. All the values are compared to normal ovary (calibrator). A matching pair is formed by mRNA from the cancer sample for a particular tissue and mRNA from the normal adjacent sample for that same tissue from the same individual.

**Table 9: Relative levels of Cln109 Expression in Individual Samples**

| **Sample ID** | **Tissue** | **Cancer** | **Matching Normal Adjacent** |
|---|---|---|---|
| Sto AC93 | Stomach 1 | 2574 | 1310 |
| Sto AC99 | Stomach 2 | 4153 | 5 |
| Sml 21XA | Small Intestine 1 | 2667 | 13663.8 |
| Sml H89 | Small Intestine 2 | 57.8 | 904.29 |
| Cln B56 | Colcn-Cecum(A)1 | 6794 | 299 |
| Cln AS45 | Colon-Ascending(A)2 | 814.6 | 105.8 |
| Cln CM67 | Colon-Cecum(B)3 | 294.6 | 36.1 |
| Cln AS67 | Colon-Ascending(B)4 | 2.2 | 26.3 |
| Cln AS43 | Colon-Ascending(C)5 | 111 | 377 |
| Cln AS46 | Colon-Ascending(C)6 | 1180 | 352 |
| Cln AS98 | Colon-Ascending(C)7 | 1075 | 92 |
| Cln AS89 | Colon-Ascending(D)8 | 14022.7 | 87.5 |
| Cln TX01 | Colon-Transverse(B)9 | 1027.6 | 282.1 |
| Cln TX89 | Colon-Transverse(B)10 | 2.5 | 23.7 |
| Cln TX67 | Colon-Transverse(C)11 | 0.1 | 72.3 |
| Cln MT38 | Colon-Splenic Flexture(M)12 | 372 | 88 |
| Cln SG89 | Colon-Sigmoid(B)13 | 179.2 | 33.4 |
| Cln SG67 | Colon-Sigmoid(C)14 | 85.0 | 94.7 |
| Cln SG33 | Colon-Sigmoid(C)15 | 5461 | 377 |
| Cln SG45 | Colon-Sigmoid(D)16 | 762.7 | 15.9 |
| Cln B34 | Colon-Rectosigmoid(A)17 | 460 | 1 |
| Cln RC67 | Colon-Rectum(B)18 | 64.5 | 136.2 |
| Cln C9XR | Colon-Rectosigmoid(C)19 | 441 | 34 |
| Cln RS45 | Colon-Rectosigmoid(C)20 | 1931 | 195 |
| Cln RC01 | Colon-Rectum(C)21 | 72.8 | 19.1 |
| Cln RC89 | Colon-Rectum(D)22 | 4.8 | 90.2 |
| Bld 46XK | Bladder 1 | 4 | 3 |
| Bld 66X | Bladder 2 | 1 | 0 |
| Bld 32XK | Bladder 3 | 0.1 | 307.6 |
| Kid 126XD | Kidney 1 | 0 | 2 |
| Kid 12XD | Kidney 2 | 3 | 16 |
| Kid 5XD | Kidney 3 | 0.0 | 0.3 |
| Kid 6XD | Kidney 4 | 18.5 | 1.2 |
| Liv 42X | Liver 1 | 21 | 0.03 |
| Liv 15XA | Liver 2 | 0.5 | 0.4 |
| Liv 94XA | Liver 3 | 0.4 | 0.0 |
| Lng AC69 | Lung 1 | 0.1 | 0 |
| Lng BR94 | Lung 2 | 3 | 0 |
| Lng 60XL | Lung 3 | 0.1 | 0 |
| Mam 59X | Mammary Gland 1 | 0 | 4 |
| Mam B011X | Mammary Gland 2 | 8 | 13 |
| Mam A06X | Mammary Gland 3 | 4.7 | 9.6 |
| Pan 71XL | Pancreas 1 | 8902.5 | 1428.2 |
| Pan 82XP | Pancreas 2 | 0.2 | 9.3 |
| Pro 12B | Prostate 1 | 9 | 20 |
| Pro 23B | Prostate 2 | 191 | 88 |
| Pro 13XB | Prostate 3 | 12 | 460 |
| Pro 34B | Prostate 4 | 3.2 | 80.4 |
| Tst 39X | Testis 1 | 29.9 | 0 |
| End 8XA | Endometrium 1 | 0.3 | 21 |
| Utr 85XU | Uterus 1 | 244.7 | 592.2 |
| Ovr 63A | Ovary 1 | 11.4 | 0 |
| Ovr A1C | Ovary 2 | 68.4 | 0 |

| | | | |
|---|---|---|---|
| 0= Negative | | | |

When matching samples were analyzed, the higher levels of expression were in small intestine, colon and stomach, showing a high degree of tissue specificity for these three colon tissues. These results confirm the tissue specificity results obtained with normal pooled samples for small intestine (Table 8). Furthermore, the level of mRNA expression in cancer samples and the isogenic normal adjacent tissue from the same individual were compared. This comparison provides an indication of specificity for the cancer stage (e.g. higher levels of mRNA expression in the cancer sample compared to the normal adjacent). Table 9 shows overexpression of CSG, Cln109 (SEQ ID NO:7) in 15 colon cancer tissues compared with their respective normal adjacent (colon samples #1, 2, 3, 6, 7, 8, 9, 12, 13, 15, 16, 17, 19, 20, and 21). There is overexpression in the cancer tissue for 68% of the colon matching samples tested (total of 22 colon matching samples). The matching sample Pan 71XL is a secondary cancer in pancreas, the primary cancer in that individual was a duodenal cancer.

Altogether, the high level of tissue specificity, plus the mRNA overexpression in more than half of the colon, stomach, and small intestine matching samples tested are demonstrative of CSG Cln109 (SEQ ID NO:7) being a diagnostic marker for colon cancer. The amino acid sequence encoded by the open reading frame of Cln109 is depicted in SEQ ID NO:10.

## Claims

1. A diagnostic method indicative of the presence of colon cancer in a patient, the method comprising:
(a) measuring levels of a colon specific gene (CSG) in a sample of cells, tissue or bodily fluid obtained from the patient; and
(b) comparing the measured levels of CSG with levels of CSG in a sample of cells, tissue or bodily fluid obtained from a control, wherein an increase in measured levels of CSG in the patient versus the CSG levels in the control is associated with the presence of colon cancer;
wherein the CSG comprises SEQ ID NO:3 or 7.

2. A diagnostic method indicative of the presence of metastatic colon cancer in a patient, the method comprising:
(a) measuring levels of CSG in a sample of cells, tissue, or bodily fluid obtained from the patient; and
(b) comparing the measured levels of CSG with levels of CSG in a sample of cells, tissue, or bodily fluid obtained from a control, wherein an increase in measured CSG levels in the patient versus the CSG levels in the control is associated with a cancer which has metastasized;
wherein the CSG comprises SEQ ID NO:3 or 7.

3. A method of staging colon cancer in a patient identified as suffering from colon cancer, the method comprising:
(a) measuring levels of CSG in a sample of cells, tissue, or bodily fluid obtained from the patient; and
(b) comparing the measured levels of CSG with levels of CSG in a sample of cells, tissue, or bodily fluid obtained from a control, wherein an increase in the measured levels of CSG versus the levels of CSG in the control is associated with a cancer which is progressing and a decrease in the measured levels of CSG versus the levels of CSG in the control is associated with a cancer which is regressing or in remission;
wherein the CSG comprises SEQ ID NO:3 or 7.

4. A method of monitoring colon cancer in a patient identified as having colon cancer that is not known to have metastasized for the onset of metastasis, the method comprising:
(a) periodically measuring CSG levels in samples of cells, tissue, or bodily fluid obtained from the patient having colon cancer that is not known to have metastasized; and
(b) comparing the periodically measured levels of CSG with levels of CSG in cells, tissue, or bodily fluid obtained from a control, wherein an increase in any one of the periodically measured levels of CSG in the patient versus the levels of CSG in the control is associated with a cancer which has metastasized;
wherein the CSG comprises SEQ ID NO:3 or 7.

5. A method of monitoring changes in a stage of colon cancer in a patient identified as suffering from colon cancer, the method comprising:
(a) periodically measuring levels of CSG in samples of cells, tissue, or bodily fluid obtained from the patient; and
(b) comparing the measured levels of CSG with levels of CSG in cells, tissue, or bodily fluid obtained from a control, wherein an increase in any one of the periodically measured levels of CSG versus levels of CSG in the control is associated with a cancer which is progressing in stage and a decrease in any one of the periodically measured levels of CSG versus the levels of CSG in the control is associated with a cancer which is regressing in stage or in remission;
wherein the CSG comprises SEQ ID NO:3 or 7.

## Revendications

1. Procédé de diagnostic indicatif de la présence de cancer du côlon dans un patient, le procédé comprenant les opérations consistant à :
(a) mesurer des taux d'un gène spécifique du côlon (CSG) dans un échantillon de cellules, de tissu ou de fluide corporel obtenu à partir du patient ; et
(b) comparer les taux mesurés de CSG avec des taux de CSG dans un échantillon de cellules, de tissu ou de fluide corporel obtenu à partir d'un témoin, une augmentation des taux mesurés de CSG dans le patient contre des taux de CSG dans le témoin étant associée avec la présence de cancer du côlon,
dans lequel le CSG comprend SEQ ID NO:3 ou 7.

2. Procédé de diagnostic indicatif de la présence de cancer de côlon métastatique dans un patient, le procédé comprenant les opérations consistant à :
(a) mesurer des taux de CSG dans un échantillon de cellules, de tissu ou de fluide corporel obtenu à partir du patient ; et
(b) comparer les taux mesurés de CSG avec des taux de CSG dans un échantillon de cellules, de tissu ou de fluide corporel obtenu à partir d'un témoin, une augmentation des taux de CSG mesurés dans le patient contre des taux de CSG dans le témoin étant associée avec un cancer qui a métastasé ;
dans lequel le CSG comprend SEQ ID NO:3 ou 7.

3. Procédé de stadification d'un cancer du côlon dans un patient identifié comme souffrant de cancer du côlon, le procédé comprenant les opérations consistant à :
(a) mesurer les taux de CSG dans un échantillon de cellules, de tissu ou de fluide corporel obtenu à partir du patient ; et
(b) comparer les taux mesurés de CSG avec des taux de CSG dans un échantillon de cellules, de tissu ou de fluide corporel obtenu à partir d'un témoin, une augmentation des taux mesurés de CSG contre des taux de CSG dans le témoin étant associée avec un cancer qui progresse et une diminution des taux mesurés de CSG contre des taux de CSG dans le témoin étant associée avec un cancer qui est en régression ou en rémission ;
dans lequel le CSG comprend SEQ ID NO:3 ou 7.

4. Procédé de surveillance pour l'apparition de métastase d'un cancer du côlon dans un patient identifié comme ayant un cancer du côlon qui n'est pas connu pour avoir métastasé, le procédé comprenant les opérations consistant à :
(a) mesurer périodiquement les taux de CSG dans des échantillons de cellules, de tissu ou de fluide corporel obtenus à partir du patient ayant un cancer du côlon qui est connu pour avoir métastasé ;
(b) comparer les taux mesurés périodiquement de CSG avec des taux de CSG dans des cellules, du tissu ou du fluide corporel obtenu à partir d'un témoin, une augmentation de l'un quelconque des taux mesurés périodiquement de CSG dans le patient contre des taux de CSG dans le témoin étant associée avec un cancer qui a métastasé ;
dans lequel le CSG comprend SEQ ID NO:3 ou 7.

5. Procédé de surveillance de changements dans un stade de cancer du côlon dans un patient identifié comme souffrant de cancer du côlon, le procédé comprenant les opérations consistant à :
(a) mesurer périodiquement les taux de CSG dans des échantillons de cellules, de tissu ou de fluide corporel obtenu à partir du patient ; et
(b) comparer les taux mesurés de CSG avec des taux de CSG dans des cellules, du tissu ou du fluide corporel obtenu à partir d'un témoin, une augmentation dans l'un quelconque des taux mesurés périodiquement de CSG contre des taux de CSG dans le témoin étant associée avec un cancer qui progresse en stade et une diminution de l'un quelconque des taux mesurés périodiquement de CSG contre des taux de CSG dans le témoin étant associée avec un cancer qui régresse en stade ou est en rémission ;
dans lequel le CSG comprend SEQ ID NO:3 ou 7.

## Patentansprüche

1. Diagnostisches Verfahren, welches das Vorliegen von Dickdarmkrebs in einem Patienten anzeigt, wobei das Verfahren umfasst:
(a) Messen der Werte eines Dickdarm-spezifischen Gens (CSG, = colon specific gene) in einer Probe aus Zellen, Gewebe oder Körperflüssigkeit des Patienten; und
(b) Vergleichen der gemessenen CSG-Werte mit CSG-Werten in einer Vergleichsprobe aus Zellen, Gewebe oder Körperflüssigkeit des Patienten, wobei ein Anstieg der gemessenen CSG-Werte im Patienten gegenüber den CSG-Werten in der Vergleichsprobe mit dem Vorliegen von Dickdarmkrebs im Zusammenhang steht;
wobei das CSG die SEQ.-ID.-Nr. 3 oder 7 umfasst.

2. Diagnostiches Verfahren, welches das Vorliegen von Dickdarmkrebs in einem Patienten anzeigt, wobei das Verfahren umfasst:
(a) Messen der CSG-Werte in einer Probe aus Zellen, Gewebe oder Körperflüssigkeit des Patienten; und
(b) Vergleichen der gemessenen CSG-Werte mit CSG-Werten in einer Vergleichsprobe aus Zellen, Gewebe oder Körperflüssigkeit des Patienten, wobei ein Anstieg der gemessenen CSG-Werte im Patienten gegenüber den CSG-Werten in der Vergleichsprobe mit Krebs im Zusammenhang steht, der metastasiert hat;
wobei das CSG die SEQ.-ID.-Nr. 3 oder 7 umfasst.

3. Verfahren zur Bestimmung des Stadiums von Dickdarmkrebs in einem Patienten, bei dem festgestellt wurde, dass er unter Dickdarmkrebs leidet, wobei das Verfahren umfasst:
(a) Messen der CSG-Werte in einer Probe aus Zellen, Gewebe oder Körperflüssigkeit des Patienten; und
(b) Vergleichen der gemessenen CSG-Werte mit CSG-Werten in einer Vergleichsprobe aus Zellen, Gewebe oder Körperflüssigkeit des Patienten, wobei ein Anstieg der gemessenen CSG-Werte gegenüber den CSG-Werten in der Vergleichsprobe mit Krebs im Zusammenhang steht, der fortschreitet, und wobei ein Abfall der gemessenen CSG-Werte gegenüber den CSG-Werten in der Vergleichsprobe mit Krebs im Zusammenhang steht, der sich zurückbildet oder in Remission befindet;
wobei das CSG die SEQ.-ID.-Nr. 3 oder 7 umfasst.

4. Verfahren zur Überwachung des Einsetzens von Metastasen bei Dickdarmkrebs in einem Patienten, bei dem festgestellt wurde, dass er Dickdarmkrebs hat, der noch nicht metastasiert hat, wobei das Verfahren umfasst;
(a) regelmäßiges Messen der CSG-Werte in Proben aus Zellen, Gewebe oder Körperflüssigkeit des Patienten, welcher Dickdarmkrebs hat, der noch nicht metastasiert hat; und
(b) Vergleichen der regelmäßig gemessenen CSG-Werte mit CSG-Werten in einer Vergleichsprobe aus Zellen, Gewebe oder Körperflüssigkeit des Patienten, wobei ein Anstieg eines der regelmäßig gemessenen CSG-Werte im Patienten gegenüber den CSG-Werten der Vergleichsprobe mit Krebs im Zusammenhang steht, der metastasiert hat;
wobei das CSG die SEQ.-ID.-Nr. 3 oder 7 umfasst.

5. Verfahren zur Überwachung der Veränderungen des Stadiums von Dickdarmkrebs in einem Patienten, bei dem festgestellt wurde, dass er unter Dickdarmkrebs leidet, wobei das Verfahren umfasst:
(a) regelmäßiges Messen der CSG-Werte in Proben aus Zellen, Gewebe oder Körperflüssigkeit des Patienten; und
(b) Vergleichen der gemessenen CSG-Werte mit CSG-Werten in einer Vergleichsprobe aus Zellen, Gewebe oder Körperflüssigkeit, wobei ein Anstieg eines der regelmäßig gemessenen CSG-Werte gegenüber den CSG-Werten in der Vergleichsprobe mit Krebs im Zusammenhang steht, der fortschreitet, und wobei ein Abfall eines der regelmäßig gemessenen CSG-Werte gegenüber den CSG-Werten in der Vergleichsprobe mit Krebs im Zusammenhang steht, der sich zurückbildet oder in Remission befindet;
wobei das CSG die SEQ.-ID.-Nr. 3 oder 7 umfasst.


### SEQUENCE LISTING

<110> Macina, Roberto A.
   Yang, Fei
   Sun, Yongming
<120> A Novel Method of Diagnosing, Monitoring and Staging Colon Cancers
<130> DEX-0035
<140>
   <141>
<150> 60/086,266
   <151> 1998-05-21
<160> 10
<170> Patentin Ver. 2.0
<210> 1
   <211> 487
   <212> DNA
   <213> Homo sapiens
<400> 1
   tctgcatctg gccctcccag tgcacctgtt caatcccagc ycctccctga cctgtacaaa 60
   tacacctgag gaccggctcg agcccagact tcctgcccct gctctgcact ctcaggtatt 120
   ccctgctctt actccaaaaa gatggaccca ggtccgaagg ggcactgcca ctgtgggggg 180
   catggccatc ctccaggtca ctgcgggcga acccctggcc atggcccagg gccctgcggg 240
   ccaccccctg gccatggccc agggccctgc gggcaacccc ctggccatgg cccagggccc 300
   tgcgggcctc cccctggcca tggcccaggt cacccacccc ctggtccaca tcactgagga 360
   agtagaagaa aacaggacac aagatggcaa gcctgagaga attgcccagc tgacctggaa 420
   tgaggcctaa accacaatct tctcttccta ataaacagcc tcctagaggc cacattctat 480
   tctttaa                                                           487
<210> 2
   <211> 739
   <212> DNA
   <213> Homo sapiens
<220>
   <221> unsure
   <222> (693)
<220>
   <221> unsure
   <222> (698)..(699)
<220>
   <221> unsure
   <222> (703)..(705)
<220>
   <221> unsure
   <222> (708)
<220>
   <221> unsure
   <222> (710)..(716)
<220>
   <221> unsure
   <222> (718)
<220>
   <221> unsure
   <222> (723)..(726)
<220>
   <221> unsure
   <222> (728)
<220>
   <221> unsure
   <222> (732)
<220>
   <221> unsure
   <222> (737)
<400> 2
   tctgaaactg tcagttccac cagcactgct tggatactgg taagtttcca gggggctgct 60
   ttgcatctga aactgtcagc cccagaatgt tgacagtcgc tctcctagcc cttctctgtg 120
   cctcagcctc tggcaatgcc attcaggcca ggtcttcctc ctatagtgga gagtatggaa 180
   gtggtggtgg aaagcgattc tctcattctg gcaaccagtt ggacggcccc atcaccgccc 240
   tccgggtccg agtcaacaca tactacatcg taggtcttca ggtgcgctat ggcaaggtgt 300
   ggagcgacta tgtgggtggt cgcaacggag acctggagga gatctttctg caccctgggg 360
   aatcagtgat ccaggtttct gggaagtaca agtggtacct gaagaagctg gtatttgtga 420
   cagacaaggg ccgctatctg tcttttggga aagacagtgg cacaagtttc aatgccgtcc 480
   ccttgcaccc caacaccgtg ctccgcttca tcagtggccg gtctggttct ctcatcgatg 540
   ccattggcct gcactgggat gtttacccca ctagctgcag cagatgctga gcctcctctc 600
   cttggcaggg gcactgtgat gaggagtaag aactccctta tcactaaccc ccatccaaat 660
   ggctcaataa aaaaatatgg ttaaggctaa aanaaaanng gannnaanan nnnnnntnca 720
   aannnnantt cncctgnta                                              739
<210> 3
   <211> 428
   <212> DNA
   <213> Homo sapiens
<220>
   <221> unsure
   <222> (391)
<400> 3
   aattgtccgg ggtcaaacag aggagagcat gaatgagagt catcctcgca agtgtgcaga 60
   gtcttttgag atgtggcatg atcgtgactc ccactgtagg cgccctaagt ttgaagggca 120
   tccccctgag tcttggaagt ggatccttgc accggtcatt ctttatatct gtgaaaggat 180
   cctccggttt taccgctccc agcagaaggt tgtgattacc aaggttgtta tgcacccatc 240
   caaagttttg gaattgcaga tgaacaagcg tggcttcagc atggaagtgg ggcagtatat 300
   ctttgttaat tgcccctcaa tctctctcct gggaatggca tccttttact ttgacctctg 360
   ctccagagga agatttcttc ttcattcata tncgagcagc aggggacttg acagaaaatc 420
   tataaggg                                                          428
<210> 4
   <211> 1347
   <212> DNA
   <213> Homo sapiens
<400> 4
   ggaaaacccc tgagcacaaa gcaagaggca tcgaagcccc ctcggggatg cccgcaagcc 60
   aacaggggtg tcgtgccgtg ggagtacttc cgcctgcgtc ctctgcggtt cagggcccca 120
   gacgagcccc agcaggccca agtcccccat gtctggggct gggaggtggc tggggcccct 180
   gcactgaggc tgcagaagtc ccagtcatct gatctgctgg aaagggagag ggagagtgtc 240
   ctgcgccggg agcaagaggt ggcagaggag cggagaaatg ctctcttccc agaggtcttc 300
   tccccaacgc cagatgagaa ctctgaccag aactccagga gctcctccca ggcatccggc 360
   atcacgggca gttactcggt gtctgagtct cccttcttca gccccatcca cctacactca 420
   aacgtggcgt ggacagtgga agatccagtg gacagtgctc ctcccgggca gagaaagaag 480
   gagcaatggt acgctggcat caacccctcg gacggtatca actcagaggt cctggaagcc 540
   atacgggtga cccgtcacaa gaacgccatg gcagagcgct gggaatcccg catctacgcc 600
   agtgaggagg atgactgagc ctcgggatgg ggcgcccacc ccctgccctg ccctgaccct 660
   cgtgggaact gccaagacca tcgccaagcc cccaccctag gaaatgggtc ctaggtccag 720
   gatccaagaa ccacagctca tctgccaaca atcccaccat gggcacattt gggactgttg 780
   ggtttttcgt ttccgttttt atcttccttt agaaatgttt ctgcctttgg ggtctaaagc 840
   ttttggggat gaaatgggga cccctgctga ttctttctgc ttctaagact ttgccaaatg 900
   ccctgggtct aagaaagaaa gagacccgct cctccacttt caggtgtaat ttgcttccgc 960
   tagtctgagg gcagaggcac cggtcaaaga gggtggcaca gatcgcagca ccttgagggg 1020
   ctgcgggtct gagggaggag acactcagct cctccctctg agaagtccca agctgagagg 1080
   ggagacctgc ccctttccaa ccctgggaaa ccatccagtc tgagggagga ggccaaactc 1140
   ccagtgctgg gggtccctgt gcagccctca aacccttcac cttggtgcac ccagccacac 1200
   ctggtggaca caaagctctc acatcgatag gatcccatga ggatggtccc cttcacctgg 1260
   gagaaaagtg acccagttta ggagctggag gggggtcttt gtcccccacc cccaaactgc 1320
   cctgaaataa acctggagtg agctgcc                                     1347
<210> 5
   <211> 1249
   <212> DNA
   <213> Homo sapiens
<220>
   <221> unsure
   <222> (1034)..(1046)
<400> 5
   ggcagagcct gcgcagggca ggagcagctg gcccactggc ggcccgcaac actccgtctc 60
   accctctggg cccactgcat ctagaggagg gccgtctgtg aggccactac ccctccagca 120
   actgggaggt gggactgtca gaagctggcc cagggtggtg gtcagctggg tcagggacct 180
   aacggcacct ggctgggacc acctcgcctt ctccatcgaa gcaggggaag tgggagcctc 240
   gagccctcgg gtggaagctg accccaagcc acccttcacc tggacaggat gagagtgtca 300
   ggtgtgcttc gcctcctggc cctcatcttt gccatagtca cgacatggat gtttattcga 360
   agctacatga gcttcagcat gaaaaccatc cgtctgccac gctggctggc ctcgcccacc 420
   aaggagatcc aggttaaaaa gtacaagtgt ggcctcatca agccctgccc agccaactac 480
   tttgcgttta aaatctgcag tggggccgcc aacgtcgtgg gccctactat gtgctttgaa 540
   gaccgcatga tcatgagtcc tgtgaaaaac aatgtgggca gaggcctaaa catcgccctg 600
   gtgaatggaa ccacgggagc tgtgctggga cagaagtcat ttgacatgta ctctggagat 660
   gttatgcacc tagtgaaatt ccttaaagaa attccggggg gtgcactggt gctggtggcc 720
   tcctacgacg atccagggac caaaatgaac gatgaaagca ggaaactctt ctctgacttg 780
   gggagttcct acgcaaaaca actgggcttc cgggacagct gggtcttcat aggagccaaa 840
   gacctcaggg gtaaaagccc ctttgagcag ttcttaaaga acagcccaga cacaaacaaa 900
   tacgagggat ggccagagct gctggagatg gagggctgca tgcccccgaa gccattttag 960
   ggtggctgtg gctcttcctc agccaggggc ctgaagaagc tcctgcctga cttaggagtc 1020
   agagcccggc aggnnnnnnn nnnnnnnnnn nnnnnntgct gcgtggaagg tgctgcaggt 1080
   ccttgcacgc tgtgtcgcgc ctctcctcct cggaaacaga accctcccac agcacatcct 1140
   acccggaaga ccagcctcag agggtccttc tggaaccagc tgtctgtgga gagaatgggg 1200
   tgctttcgtc agggactgct gacggctggt cctgaggaag gacaaactg            1249
<210> 6
   <211> 1220
   <212> DNA
   <213> Homo sapiens
<400> 6
   gctttctgca cctcattcca catcaggagc gtttttggag aaagctgcac tctgttgagc 60
   tccagggcgc agtggaggga gggagtgaag gagctctctg tacccaagga aagtgcagct 120
   gagactcaga caagattaca atgaaccaac tcagcttcct gctgtttctc atagcgacca 180
   ccagaggatg gagtacagat gaggctaata cttacttcaa ggaatggacc tgttcttcgt 240
   ctccatctct gcccagaagc tgcaaggaaa tcaaagacga atgtcctagt gcatttgatg 300
   gcctgtattt tctccgcact gagaatggtg ttatctacca gaccttctgt gacatgacct 360
   ctgggggtgg cggctggacc ctggtggcca gcgtgcacga gaatgacatg cgtgggaagt 420
   gcacggtggg cgatcgctgg tccagtcagc agggcagcaa agcagtctac ccagaggggg 480
   acggcaactg ggccaactac aacacctttg gatctgcaga ggcggccacg agcgatgact 540
   acaagaaccc tggctactac gacatccagg ccaaggacct gggcatctgg cacgtgccca 600
   ataagtcccc catgcagcac tggagaaaca gctccctgct gaggtaccgc acggacactg 660
   gcttcctcca gacactggga cataatctgt ttggcatcta ccagaaatat ccagtgaaat 720
   atggagaagg aaagtgttgg actgacaacg gcccggtgat ccctgtggtc tatgattttg 780
   gcgacgccca gaaaacagca tcttattact caccctatgg ccagcgggaa ttcactgcgg 840
   gatttgttca gttcaggcta tttaataacg agagagcagc caacgccttg tgtgctggaa 900
   tgagggtcac cggatgtaac actgagcacc actgcattgg tggaggagga tactttccag 960
   aggccagtcc ccagcagtgt ggagattttt ctggttttga ttggagtgga tatggaactc 1020
   atgttggtta cagcagcagc cgtgagataa ctgaggcagc tgtgcttcta ttctatcgtt 1080
   gagagttttg tgggagggaa cccagacctc tcctcccaac catgagatcc caaggatgga 1140
   gaacaactta cccagtagct agaatgttaa tggcagaaga gaaaacaata aatcatattg 1200
   actcaaaaaa aaaaaaaaag                                             1220
<210> 7
   <211> 2796
   <212> DNA
   <213> Homo sapiens
<400> 7
   cggctcgagg gacaggatga ggcccggcct ctcatttctc ctagcccttc tgttcttcct 60
   tggccaagct gcaggggatt tgggggatgt gggacctcca attcccagcc ccggcttcag 120
   ccctttccca ggtgttgact ccagctccag cttcagctcc agctccaggt cgggctccag 180
   ctccagccgc agcttaggca gcggaggttc tgtgtcccag ttgttttcca atttcaccgg 240
   ctccgtggat gaccgtggga cctgccagtg ctctgtttcc ctgccagaca ccacctttcc 300
   cgtggacaga gtggaacgct tggaattcac agctcatgtt ctttctcaga agtttgagaa 360
   agaactttcc aaagtgaggg aatatgtcca attaattagt gtgtatgaaa agaaactgtt 420
   aaacctaact gtccgaattg acatcatgga gaaggatacc atttcttaca ctgaactgga 480
   cttcgagctg atcaaggtag aagtgaagga gatggaaaaa ctggtcatac agctgaagga 540
   gagttttggt ggaagctcag aaattgttga ccagctggag gtggagataa gaaatatgac 600
   tctcttggta gagaagcttg agacactaga caaaaacaat gtccttgcca ttcgccgaga 660
   aatcgtggct ctgaagacca agctgaaaga gtgtgaggcc tctaaagatc aaaacacccc 720
   tgtcgtccac cctcctccca ctccagggag ctgtggtcat ggtggtgtgg tgaacatcag 780
   caaaccgtct gtggttcagc tcaactggag agggttttct tatctatatg gtgcttgggg 840
   tagggattac tctccccagc atccaaacaa aggactgtat tgggtggcgc cattgaatac 900
   agatgggaga ctgttggagt attatagact gtacaacaca ctggatgatt tgctattgta 960
   tataaatgct cgagagttgc ggatcaccta tggccaaggt agtggtacag cagtttacaa 1020
   caacaacatg tacgtcaaca tgtacaacac cgggaatatt gccagagtta acctgaccac 1080
   caacacgatt gctgtgactc aaactctccc taatgctgcc tataataacc gcttttcata 1140
   tgctaatgtt gcttggcaag atattgactt tgctgtggat gagaatggat tgtgggttat 1200
   ttattcaact gaagccagca ctggtaacat ggtgattagt aaactcaatg acaccacact 1260
   tcaggtgcta aacacttggt ataccaagca gtataaacca tctgcttcta acgccttcat 1320
   ggtatgtggg gttctgtatg ccacccgtac tatgaacacc agaacagaag agatttttta 1380
   ctattatgac acaaacacag ggaaagaggg caaactagac attgtaatgc ataagatgca 1440
   ggaaaaagtg cagagcatta actataaccc ttttgaccag aaactttatg tctataacga 1500
   tggttacctt ctgaattatg atctttctgt cttgcagaag ccccagtaag ctgtttagga 1560
   gttagggtga aagagaaaat gtttgttgaa aaaatagtct tctccactta cttagatatc 1620
   tgcaggggtg tctaaaagtg tgttcatttt gcagcaatgt ttaggtgcat agttctacca 1680
   cactagagat ctaggacatt tgtcttgatt tggtgagttc tcttgggaat catctgcctc 1740
   ttcaggcgca ttttgcaata aagtctgtct agggtgggat tgtcagaggt ctaggggcac 1800
   tgtgggccta gtgaagccta ctgtgaggag gcttcactag aagccttaaa ttaggaatta 1860
   aggaacttaa aactcagtat ggcgtctagg gattctttgt acaggaaata ttgcccaatg 1920
   actagtcctc atccatgtag caccactaat tcttccatgc ctggaagaaa cctggggact 1980
   tagttaggta gattaatatc tggagctcct cgagggacca aatctccaac ttttttttcc 2040
   cctcactaca cctggaatga tgctttgtat gtggcagata agtaaatttg gcatgcttat 2100
   atattctaca tctgtaaagt gctgagtttt atggagagag gcctttttat gcattaaatt 2160
   gtacatggca aataaatccc agaaggatct gtagatgagg cacctgcttt ttcttttctc 2220
   tcattgtcca ccttactaaa agtcagtaga atcttctacc tcataacttc cttccaaagg 2280
   cagctcagaa gattagaacc agacttacta accaattcca ccccccacca acccccttct 2340
   actgcctact ttaaaaaaat taatagtttt ctatggaact gatctaagat tagaaaaatt 2400
   aattttcttt aatttcatta tggactttta tttacatgac tctaagacta taagaaaatc 2460
   tgatggcagt gacaaagtgc tagcatttat tgttatctaa taaagacctt ggagcatatg 2520
   tgcaacttat gagtgtatca gttgttgcat gtaatttttg cctttgttta agcctggaac 2580
   ttgtaagaaa atgaaaattt aatttttttt tctaggacga gctatagaaa agctattgag 2640
   agtatctagt taatcagtgc agtagttgga aaccttgctg gtgtatgtga tgtgcttctg 2700
   tgcttttgaa tgactttatc atctagtctt tgtctgtttt tcctttgatg ttcaagtcct 2760
   agtctatagg attggcagtt taaatgcttt actccc 2796
<210> 8
   <211> 2331
   <212> DNA
   <213> Homo sapiens
<220>
   <221> unsure
   <222> (675)
<400> 8
   tttatcacgg gctcaactgc aacaaaacac ttccttgaca gctccacaaa ctcaggccac 60
   agtgaggaat caacaatatt ccacagcagc ccagatgcaa gtggaacaac accctcatct 120
   gcccactcca caacctcagg tcgtggagaa tctacaacct cacgcatcag tccaggctca 180
   actgaaataa caacgttacc tggcagtacc acaacaccag gcctcagtga ggcatctacc 240
   accttctaca gtagccccag atcaccagac caaacactct cacctgccag catgagaagc 300
   tccagcatca gtggagaacc caccagcttg tatagccaag cagagtcaac acacacaaca 360
   gcgttccctg ccagcaccac cacctcaggc ctcagtcagg aatcaacaac tttccacagt 420
   aagccaggct caactgagac aacactgtcc cctggcagca tcacaacttc atcttttgct 480
   caagaattta ccacccctca tagccaacca ggctcagctc tgtcaacagt gtcacctgcc 540
   agcaccacag tgccaggcct tagtgaggaa tctaccacct tctacagcag cccaggctca 600
   actgaaacca cagcgttttc tcacagcaac acaatgtcca ttcatagtca acaatctaca 660
   cccttccctg acagnccagg cttcactcac acagtgttac ctgccaccct cacaaccaca 720
   gacattggtc aggaatcaac agccttccac agcagctcag acgcaactgg aacaacaccc 780
   ttacctgccc gctccacagc ctcagacctt gttggagaac ctacaacttt ctacatcagc 840
   ccatccccta cttacacaac actctttcct gcgagttcca gcacatcagg cctcactgag 900
   gaatctacca ccttccacac cagtccaagc ttcacttcta caattgtgtc tactgaaagc 960
   ctggaaacct tagcaccagg gttctgccag gaaggacaaa tttggaatgg aaaacaatgc 1020
   gtctgtcccc aaggctacgt tggttaccag tgcttgtccc ctctggaatc cttccctgta 1080
   gaaaccccgg aaaaactcaa cgccacttta ggtatgacag tgaaagtgac ttacagaaat 1140
   ttcacagaaa agatgaatga cgcatcctcc caggaatacc agaacttcag taccctcttc 1200
   aagaatcgga tggatgtcgt tttgaagggc gacaatcttc ctcagtatag aggggtgaac 1260
   attcggagat tgctcaacgg tagcatcgtg gtcaagaacg atgtcatcct ggaggcagac 1320
   tacactttag agtatgagga actgtttgaa aacctggcag agattgtaaa ggccaagatt 1380
   atgaatgaaa ctagaacaac tcttcttgat cctgattcct gcagaaaggc catactgtgc 1440
   tatagtgaag aggacacttt cgtggattca tcggtgactc cgggctttga cttccaggag 1500
   caatgcaccc agaaggctgc cgaaggatat acccagttct actatgtgga tgtcttggat 1560
   gggaagctgg cctgtgtgaa caagtgcacc aaaggaacga agtcgcaaat gaactgtaac 1620
   ctgggcacat gtcagctgca acgcagtgga cccccgctgc ctgtgcccaa atacgaacac 1680
   acactggtac tggggagaga cctgtgaatt caacatcgcc aagagcctcg tgtatgggat 1740
   cgtgggggct gtgatggcgg tgctgctgct cgcattgatc atcctaatca tcttattcag 1800
   cctatcccag agaaaacggc acagggaaca gtatgatgtg cctcaagagt ggcgaaagga 1860
   aggcacccct ggcatcttcc agaagacggc catctgggaa gaccagaatc tgagggagag 1920
   cagattcggc cttgagaacg cctacaacaa cttccggccc accctggaga ctgttgactc 1980
   tggcacagag ctccacatcc agaggccgga gatggtagca tccactgtgt gagccaacgg 2040
   gggcctccca ccctcatcta gctctgttca ggagagctgc aaacacagag cccaccacaa 2100
   gcctccgggg cgggtcaaga ggagaccgaa gtcaggccct gaagccggtc ctgctctgag 2160
   ctgacagact tggccagtcc cctgcctgtg ctcctgctgg ggaaggctgg gggctgtaag 2220
   cctctccatc cgggagcttc cagactccca gaagcctcgg cacccctgtc tcctcctggg 2280
   tggctcccca ctctggaatt tccctaccaa taaaagcaaa tctgaaagct c          2331
<210> 9
   <211> 909
   <212> DNA
   <213> Homo sapiens
<400> 9
   gaggaggtgg gcgccaacag acaggcgatt aatgcggctc ttacccaggc aaccaggact 60
   acagtataca ttgtggacat tcaggacata gattctgcag ctcgggcccg acctcactcc 120
   tacctcgatg cctactttgt cttccccaat gggtcagccc tgacccttga tgagctgagt 180
   gtgatgatcc ggaatgatca ggactcgctg acgcagctgc tgcagctggg gctggtggtg 240
   ctgggctccc aggagagcca ggagtcagac ctgtcgaaac agctcatcag tgtcatcata 300
   ggattgggag tggctttgct gctggtcctt gtgatcatga ccatggcctt cgtgtgtgtg 360
   cggaagagct acaaccggaa gcttcaagct atgaaggctg ccaaggaggc caggaagaca 420
   gcagcagggg tgatgccctc agcccctgcc atcccaggga ctaacatgta caacactgag 480
   cgagccaacc ccatgctgaa cctccccaac aaagacctgg gcttggagta cctctctccc 540
   tccaatgacc tggactctgt cagcgtcaac tccctggacg acaactctgt ggatgtggac 600
   aagaacagtc aggaaatcaa ggagcacagg ccaccacaca caccaccaga gccagatcca 660
   gagcccctga gcgtggtcct gttaggacgg caggcaggcg caagtggaca gctggagggg 720
   ccatcctaca ccaacgctgg cctggacacc acggacctgt gacaggggcc cccactcttc 780
   tggacccctt gaagaggccc taccacaccc taactgcacc tgtctccctg gagatgaaaa 840
   tatatgacgc tgccctgcct cctgcttttg gccaatcacg gcagacaggg gttggggaaa 900
   tattttatt                                                         909
<210> 10
   <211> 510
   <212> PRT
   <213> Homo sapiens
<400> 10